# EUROPEAN PATENT SPECIFICATION

(11) **EP 3 930 807 B1**
(45) Date of publication and mention of the grant of the patent: **08.11.2023**
(21) Application number: 19824275.2
(22) Date of filing: 12.12.2019
(51) Int. Cl.: A61M 16/00, G01F 1/74, A61M 15/00

(54) **A METHOD AND A DEVICE FOR ESTIMATING AN AMOUNT OF A POWDER SHAPED MATERIAL PASSING A BEND IN A FLOW CHANNEL**
VERFAHREN UND VORRICHTUNG ZUM SCHÄTZEN EINER MENGE EINES PULVERFÖRMIGEN MATERIALS, DAS DURCH DIE BIEGUNG IN EINEM STRÖMUNGSKANAL GEHT
PROCÉDÉ ET DISPOSITIF D'ESTIMATION D'UNE QUANTITÉ D'UN MATÉRIAU POUDREUX PASSANT PAR UN COUDE DANS UN CANAL D'ÉCOULEMENT

(30) Priority: 27.02.2019 DK PA201970124
(43) Date of publication of application: 05.01.2022
(73) Proprietor: NuvoAir AB, 114 57 Stockholm (SE)
(72) Inventor: CONSOLI, Lorenzo, 11452 Stockholm (SE); AIDEHAG, Fredrik, 11756 Stockholm (SE); BOSAEUS, Linus, 11418 Stockholm (SE)
(74) Representative: Inspicos P/S
(86) International application number: PCT/EP2019/084902
(87) International publication number: WO 2020/173595

(56) References cited:
- EP-A1- 3 338 842
- WO-A1-2016/165029
- US-A- 5 842 468
- US-A1- 2015 273 165
- US-A1- 2018 161 530

## Description

The present invention relates to the estimation of the amount of a powder material passing a bend in a flow channel and in particular to how much drug is actually inhaled and not deposited in the mouth or throat of a person.

Inhalation technology may be seen in the known inhalation trainers, dry powder inhalers (DPI), metered-dose inhaler (pMDI), nebulisers, spirometers, or the like, such as Turbuhaler. Such technology may be seen in e.g. US5842468, EP0667168, US2010/0242955, CN1162271, US2003/0136400, EP3338842, US2018/161530, WO2016/165029, US2015/273165 and US4984158.

It is an objective of embodiments of the invention to provide an improved method of determining how much of a powder shaped material passes a bend in a flow channel. An interesting embodiment is for an inhaler or inhalation training for a person, where it is interesting to ensure that as much as possible of the material, here usually in the form of a drug, actually reaches the lungs of the person and thus passes the throat of the person.

It is known that the air flow through the bend of the throat should be suitable, as a too low air velocity will allow the drug to settle in the mouth of the person and that the drug at a too high velocity will impact on the walls of the throat and not be able to follow the air flow toward the lungs.

In a first aspect, the invention relates to a method according to claim 1.

In the present context, material is powder shaped. Powder shaped materials usually are solid or porous materials with a particle size sufficiently small for it to be collected by an entrained in a gas flow. In some embodiments, the gas flow may be an air flow generated by a person inhaling. Thus, the air flow may depend on parameters of the material. For DPIs, flow rates are often about 30-120L/m.

Powder-shaped materials may generally have any particle size. However, particle sizes (mean diameter) of 100µm or less are usual, such as 10 µm or less, such as 6µm or less or 8-4pm or 6-4µm. For DPIs, the diameter may be in the range of around 5.5 µm.

The weight of the individual particles of interest may be 10-1000pg. This weight is often not mentioned, but a usual density is around that of sugar, as sugar molecules often are used as carrier material in the drugs.

The size of the powder may depend on the actual material and/or the manner in which it is generated. Different particle sizes and/or distributions may be desired for different purposes, such as different person types (gender, age, physical condition, illness or the like).

In some situations, a powder is sticky/moist in the sense that if it impacts on the flow channel wall, it will have a tendency to adhere to the wall and thus not be transported further along the flow channel even though the gas keeps flowing therein. In other situations, the flow channel walls are sticky/moist and thus tend to prevent the powder from flowing with the gas.

The flow channel often has an inlet and an exit. Usually, the flow channel between the input and exit is gas tight so that no gas can enter or exit the flow channel between the input and exit. The flow channel may be branched if desired.

The flow channel may have the same cross section or cross sectional area along its length. Alternatively, the cross sectional shape and/or area may vary.

The flow channel has a bend. In this context, a bend is a portion of the flow channel in which the direction of the gas flowing therein changes a general direction of the flow. Clearly, multiple bends may be provided if desired.

In one situation, the cross sectional area at or in the bend is of interest, as the bend forms a constriction or obstacle which not only alters the direction of the gas but also of the material. Thus, if the material is moving too fast, it may not be re-directed to the same degree and thus impact on the bend of the flow channel. Clearly, the flow channel may comprise additional obstacles, such as splits dividing the flow channel into separate flow channels. Then, it may be desired to maintain a desired flow even after the material has negotiated the (first) bend.

The flow channel may have a first flow channel portion before the bend and a second flow channel portion after the bend. In a preferred embodiment, the flow channel has a first direction (in a first flow channel portion) before the bend and a second direction (in a second flow channel portion) after the bend. Then, the angle between the first and second directions may be at least 5 degrees, such as at least 10 degrees, such as at least 20 degrees, such as at least 45 degrees, such as at least 70 degrees, such as at least 80 degrees. The angle between the first and second directions may be in the interval of 45 and 135 degrees if desired.

The bend may also have any desired curvature. In this situation, the curvature may be a mean curvature of the bend portion of the flow channel, such as of a centre thereof, between the first and second flow channel portions. The curvature may be determined by projecting the flow channel, or at least the first and second flow channel portions and the bend on to a plane and determining a radius/diameter of a circle to which the first and second flow channel portions are tangents and where the flow channel portions or borders between the bend and the first flow channel and the second flow channel portion, respectively, are positioned on the circle.

Clearly, the larger the bend angle and the smaller the bend radius (or an equivalent thereof) when the bend is not shaped as a portion of a circle, and the smaller the cross-sectional area of the flow path, the more difficult will it be for the material to pass the bend without impacting on the bend walls.

In some embodiments, the curvature is 1-10cm.

In the present context, the material passes the bend, if it is entrained in the gas flow also after negotiating the bend. In some situations, the material passing the bend has not impacted on walls of the flow channel but preferably stayed entrained in the gas flow before, during and after passing the bend.

A container may be any type of element capable of holding the material. The container may be a blister pack or any other type of container holding a single, metered dose of the material. The container may be capable of outputting a metered or predetermined quantity of the material. The container may comprise a multiple of the predetermined quantity, usually called multiple doses. Thus, the container may comprise a metering element capable of metering a quantity of the material and outputting only this portion of the material.

In another situation, the container comprises only the predetermined quantity of the material. Then, this material may become available by opening the container. In this situation, the container may be a single-use container such as a blister pack.

The opening of the container makes the material therein available to the air flow so that the material may be carried out of the container by the air. This opening may be a breaking/crushing of the container, or an element may be brought to penetrate the container so as to allow the gas to flow through the container. Often, the opening provides a number, such as at least two, openings, where the gas enters the container through one and exits through another, so that the air may flow through the container and thereby provide an air flow in the container, to entrain the material therein and carry it out of the container. The air flow through the container may create a vortex in the container, which vortex may stir up the material therein and thus entrain the material in the gas.

The gas flow is obtained by a person inhaling, so that at least a part of the flow channel, preferably comprising the bend, is formed by the person's mouth, throat, tracheal tube and/or lungs.

The predetermined quantity of the material is released into the flow channel in which the gas flow is generated. Thus, the material will then be entrained in the flow and thus be carried with the gas through the flow channel and thus the bend. Then, it is desired to determine how much of the material actually passes the bend/restriction.

Clearly, the rate of release indicates how much material is entrained in the gas at a particular point in time. Then, the rate of release may be used for determining an amount of material presently entrained in the gas as well as the amount of material presently being in the vicinity of the bend, for example.

The releasing of the material into the flow channel may take place over a longer period of time. Powder collected by an air flow through a blister may be removed rather slowly, as the air flow will entrain the material depending on the velocity of the air flow.

In one situation, the material is initially stationary in relation to the flow channel. In one situation, the material is present in a container, such as a non-pressurized container, which is opened/ruptured to allow access to the material. Then, the entraining of the material into the gas flow may be achieved by directing the gas flow, or at least a portion thereof, through the container. The gas flow in the container may create a vortex or flow pattern inside the container so that the material therein is entrained in the flow and thus will be guided out of the container and into the remaining portion of the flow path. In this situation, the gas flow both enters and exits the container in order to entrain the material therein in the gas flow.

The first time interval may be from a first point in time at which material is made available by the container or when at least 2% such as at least 5%, such as at least 25% of the material is or has been available and until a second point in time at which no more material is available or at which at least 80%, such as at least 90%, such as at least 95% of the material has been entrained in the gas. The first time interval may be extended, as the rate of release will naturally drop, when no more material is available from or in the container.

Alternatively, the first time interval may start when a container is opened or handled by an operator in preparation for opening, such as a priming of an opening mechanism or a shaking of the container to mix the contents therein.

During the first time interval, or at least as long as a sufficient amount of the material is available from or in the container, information relating to the gas flow is obtained. This information may be a flow velocity, a volume per unit time or the like.

The gas flow may be determined at any position along the flow channel, especially if the flow channel is more or less gas tight along its length, so that the same volume of gas passes each cross sectional plane of the flow channel. Clearly, a change in cross sectional area of the flow channel will affect the flow velocity, as will turbulence and the like, but this is well-known and may be taken into account in the calculation. It is noted that the release of the quantity of the powder may be accompanied by additional gas (such as a propellant), so that the flow in some portions of the flow channel may differ from that at other positions. In other situations, only a part of the flow passes through the container, where other parts pass outside of the container. This may be taken into account by the calculations. Often, it is desired to measure the flow at the flow channel entrance, as other portions of the flow channel may be less easily accessible. Then, a percentage of the measured flow may pass through the container. This percentage may be determined by the apparatus, such as relative sizes and dimensions of the flow path and the container openings and the like.

Actually, as it will take time for the material released to reach the bend and any additional, more distal obstacles, it may be desired to determine the flow for a larger period of time. From the flow, a time delay may be determined from the release of the material and to the material reaches the bend - and the delay until that material reaches other, more distal, obstacles. For example, an undesired flow rate at a point in time where a portion of the material has negotiated the bend but is still in the flow channel, such as at a more distal obstacle, will increase the loss of material at that position. Then, the total amount of material actually negotiating the obstacles in the flow channel may be determined on the basis of the rate of release, the flow rate and the positions of the bend/obstacles along the flow channel.

For inhalers, it is desired to ensure that the inhalation is sufficiently "deep" so that the material is transported all the way to the lungs. However, it is desired that the flow stays within the desired interval during this transport, as a too low or too high flow, for example, may cause a loss of material even if the material has passed the throat - by impact on the tracheal tube wall.

Alternatively, the time interval may end when the flow is below a threshold value. However, situations exist where a user performs multiple inhalations in order to inhale all of a drug. Thus, if, for example, no more than 80% of the material, such as no more than 70% of the material, has been entrained in the gas flow when the flow falls below the threshold value, the system may stay active to be able to detect a second inhalation. In this context, the calculation may be performed during a period of time where the flow is below the threshold, or it may be resumed when the next inhalation is detected where the rate of release is then determine on the basis of the partly inhaled material dose.

The amount of material passing the bend depends on material parameters as well as parameters of the gas flow. The gas flow may vary over time, as may the amount of material in the gas flow, whereby the determination of the total amount of material actually passing the bend may be performed in steps in which the parameters can be assumed constant.

Naturally, the points in time may be determined or defined when a sufficiently large change is seen in the rate of delivery and/or the flow. Alternatively, the points in time may be equidistant, such as with a frequency of 2Hz or more, such as 5Hz or more, such as 10Hz or more, such as 25Hz or more.

At a number of points in time, such as as long material is available and has not yet passed the bend (or been retained by the flow channel), the actual rate of release is determined, so that it is known how much material is presently fed into the gas flow. Clearly, not all of the material needs be entrained in the gas flow at the same time. The material may be dispensed or released over time, where the rate of release of material then relates to how much material is presently "picked up" by the gas flow. In this context, the "rate of release" is the amount, per unit time, of the material which is "picked up" or becomes entrained in the gas flow.

Then, the calculation at a point in time is able to determine an amount of the presently available material which actually passes the bend - and then the total amount of material having passed the bend may be estimated.

Clearly, the amount of material, per unit time, passing the bend will depend on the quantity of material available to or entrained in the gas flow but also other parameters, such as the gas flow, as will be described below.

As mentioned, as the gas flow may change and/or the rate of release of the material may change, so the calculation is preferably divided up into calculations at different points of time, so that the actual rate of release and the actual flow may be used in the calculation for that point in time to determine or estimate how much material, under these circumstances, will pass the bend.

This calculation may be considered valid for a period of time. Then, a parameter may have changed so much that a new situation exists and another calculation be required. Naturally, the calculations may, as is described further below, be performed at fixed points in time, such as periodically.

Finally, the results of the individual calculations may be combined to arrive at an estimate of the total amount of the material which has passed the bend.

Clearly, other manners may exist of determining the present rate of release of the material so that the total amount of material passing the bend may be determined in other manners.

The releasing step comprises opening the container, and the step of providing the gas flow comprises providing at least a part of the gas flow through the container. The container may be in the form of a non-pressurized single-use container, such as a blister pack, each container having a quantity of the material or may be a refillable container.

Then, the container may be opened, such as punctured or ruptured so that at least a portion of the gas of the gas flow passes through the container. This portion of the gas flow will pick up a portion of the material and transfer this portion out of the container and toward the bend.

Clearly, the gas flow through the container may cause a vortex or chaotic flow pattern in the container so that the material therein will be entrained in the flow and thus be guided out of the container. This is in contrast to situations where the material is forced into the gas flow, such as from a pressurized container.

Not all of the material in the container need be entrained at the same time. The amount of material entrained in the gas in the container, or the concentration of entrained material in the gas in the container, may vary over time, such as with the total quantity of material in the container. Then, the calculation of the rate of release is preferably based also on the determined amount of material remaining in the container.

Also, the amount of material entrained in the gas in the container may depend on the flow rate/velocity of the gas in the flow path or in the container. A more gentle flow may "stir up" or entrain less material. It may allow already entrained powder to re-settle on the bottom of the container. Thus, the step of determining the rate of release of the material into the flow channel may comprise basing the determination of the rate of release on the actual flow of the gas in the flow channel.

In one situation the calculation comprises also determining, at each point in time, an amount of the material remaining in the container.

At each point in time, the rate of release may then be determined, so that the amount of material available and/or left in the container may be determined. This may be used for the calculation of the rate of release for a next point in time.

Thus, for a non-pressurized container in which gas flows, the concentration of material in the gas in the container will often depend on the flow of the gas. This concentration of material thus also is seen in the gas leaving the container. This may determine the rate of release of the material. Over time, less material will be present in the container, so that the concentration, for the same flow, of material in the gas will decrease. Thus, for the same flow, a smaller rate of release will be seen over time. Thus, the rate of release may be represented by a graph displaying the amount of material released over time.

In one embodiment, the calculation of the amount of the released material having passed the bend is based on a comparison of the actual flow in the channel to a first flow interval. In some embodiments, a too low flow will allow the material to settle in the flow channel before reaching the bend. On the other hand, a too large flow could result in material depositing in the bend due to it having a too high velocity to be re-directed sufficiently by the gas. This "sweet spot" of flow allowing the material to negotiate the bend may be defined by the interval. Clearly, the upper and lower limits of the interval may depend on a number of factors, such as the size/weight/shapes of the powder.

Clearly, a time delay may exist from release of material until that material reaches the bend. Thus, the calculation may take this into account so that the calculation relates to material released at one point in time but reaches the bend at a later point in time. Clearly, this may be expanded to flow channels having multiple bends or obstacles, where material negotiating the bend then later reaches another obstacle, where the flow at that point in time then may determine how much of that material negotiates the next obstacle.

In general, the calculated amount of material having passed the bend is preferably determined on the basis of the determined rate of release. Especially, it is preferred that the amount is determined from both the rate of release, indicating the amount of material presently carried by the gas, as well as the flow rate, such as the flow velocity, as this latter parameter may define the portion of the material in the gas will actually negotiate the bend.

Thus, the combination of these parameters may give an indication of the amount of material negotiating the bend.

In this regard, it is noted that the predetermined quantity of material will be a limited amount of the material. Especially in the situation where the material is a drug, it is important that the person does not get more drug than intended - as well as to ensure that the person then actually inhales all of the drug.

As described, it may be important that the flow is within a desired interval in order to ensure that any material in the gas actually negotiates the bend. Then, when the quantity of material is limited, any material lost due to undesired flow, will be lost. Any subsequent, correct flow will not change this, as the quantity of material is limited.

Therefore, the calculation may be terminated when a predetermined amount of the material, such as all of the material or at least 80% thereof, or at least 90% thereof or at least 95% thereof, has been released into the flow channel. As no more material, or only a small amount of material, is available, this may be assumed to not influence the total amount of material having negotiated the bend.

As mentioned above, a second flow or inhalation may take place, so that this may be added to the first one in the final calculation.

Clearly, the method may comprise also outputting the result of the calculation and/or other information, such as the rate of release, the amount, over time, of material passing the bend, the amount of material left in the container, when no more material is available, or the like. This outputting may be on any interface and using any type of outputting, such as to a monitor, LEDs mounted visible to an operator, to a storage for later retrieval or the like.

The calculation may start when a gas flow, such as an air flow, is detected in the flow path and/or when material has been made available, such as when a dose is dispensed or a container is opened. Alternatively, the calculation may start when the container or system is handled for preparation of release, such as shaking or priming.

A second aspect of the invention relates to a device according to claim 8.

Naturally, all embodiments, situations and other statements made in relation to the first aspect are also valid for the second aspect of the invention.

The present device may be a single element or product, such as a handheld product. Alternatively, the device may comprise multiple, separate elements. In one situation, the calculating means may be separate or separatable from the container and/or the sensor. Also, the container may be replaceable or removable from the sensor and/or the flow channel. In addition, the container and releasing means may form part of a usual inhaler, where the sensor and the calculating means may form part of a separate element attachable to the inhaler. Then, the flow channel forms, in addition to e.g. the throat etc. of a person, part of the inhaler and part of the separate element with the sensor.

In this connection, the flow channel or parts thereof may be formed by one or more elements forming a channel which may, apart from an entrance and an exit, be more or less gas tight. Clearly, leaks may be present as long as they do not leak too much of the material or disturb the flow measurement too much. In a particular example, the flow channel is formed by the mouth, throat and potentially also lungs of a human. As mentioned above, all of or part of the flow channel may be through the container.

The bend, as described above, forms a constriction or obstacle which the material is desired to negotiate.

The sensor may be positioned in the flow channel or be provided so that it is able to determine a gas flow in the flow channel. This flow may be a flow velocity or a flow volume of the gas, for example. Multiple types of flow sensors are known, such as pressure sensors, pressure difference sensors, rotating fans, micro thermal flow through principle and the like. Any type of sensor may be used.

Clearly, the flow channel may be formed by separate elements, such as along its length, and the flow sensor may be positioned in one such element.

The device has releasing means for releasing, during a first time interval, the predetermined quantity of the material into the gas flow in the flow channel. The releasing means may be a part of the container, such as if the container comprises more than the predetermined quantity of the material. In this manner, the predetermined quantity may be output from or released from the container by the releasing means.

On the other hand, if the container comprises only the predetermined quantity of the material, the material may be released by opening the container. The releasing means thus may be means for opening the container, such as by cutting/piercing/puncturing or the like. As described above and below, the container may be unpressurized so that upon opening, the material may not automatically be output or ejected. Then, the gas flow may be used for transporting the material out of the container and toward the bend.

The calculating means may be attached to, or form part of, the device or may be remote therefrom. Cloud computing, remote servers and the like are widely used and are equally applicable in this set-up. The calculating means may comprise one or more processors, controllers, ASICs, FPGAs or combinations thereof. Communication elements may be provided for providing communication between controllers, processors and the like as well as between the calculating means and the sensor and any optional outputting elements which may be provided for outputting a result of the calculation and potentially also other information, as will be described below.

The calculating means are configured to, such as programmed to, calculate, for a number of points of time during the first time interval:
- a rate of release of the material into the flow channel and
- an amount of the released material having passed the bend.

As mentioned above, the rate of release may relate to the manner in which the material is made available. For some container types, the material is made available over a longer period of time. For some container types, the rate of release depends on the gas flow and/or the amount of material left in the container. Thus, the rate of release for some container types may be pre-programmed or known so that the rate of release at any point in time may be determined from this knowledge. Thus, the rate of release may depend on the flow, such as the overall flow, when a known percentage of this flow passes through the container. Clearly, the percentage may be defined by dimensions of the device - and may depend on the flow rate.

Thus, the rate of release is preferably determined at the actual point in time. Thus, the rate of release may differ between points of time. As described above, the ratio of the material within the flow path travelling toward the bend to the amount of that material actually negotiating the bend will depend on the flow at that point in time. The amount of material travelling toward the bend will depend on the rate of release.

As mentioned, the device may comprise outputting elements such as for outputting information from the calculation. The total amount of the material calculated to have negotiated the bend may be output, as may the amount of that material released over time. The flow may be illustrated over time, as may information as to when a predetermined amount, such as 100% or substantially 100%, of the material has been entrained in the gas flow.

Clearly, the calculation may be ended, when no more material is released. This may be due to the container being empty, the gas flow being reduced to a low value (or stopping), or the like. Obviously, a perfect flow in the flow path brings about no increase in the amount of the material passing the bend, when there is no more material to transport by the flow. The above second inhalation may, however, be detected and added to the calculation.

According to the invention, the container is positioned in or at a portion of the flow channel, so that an inner space of the container, when the container is opened, forms part of the flow channel. Thus, when the container is opened, the material is automatically provided in the flow channel. In this embodiment, the container is usually not pressurized and the material is a powder. Before opening the container, the flow channel may be blocked by the container.

A third aspect, not forming part of the invention, relates to a device for estimating an amount of a powder shaped material passing a bend in a flow channel, the device comprising:
- a sensor for determining a flow of a gas through the flow channel,
- storing means holding information relating to:
   - a percentage of the gas flow which travels through a container holding the material,
   - a rate of release of the material as a function of the gas flow in the channel and
   - a percentage of dispensed material passing the bend as a function of the gas flow in the channel and
- calculating means for determining the amount of the material passing the bend on the basis of the actual gas flow in the flow channel, the rate of dispensing at that flow and the percentage at that flow.

In this respect, the situations and considerations described in relation to the other aspects of the invention are equally valid in the third aspect.

The present aspect, however may relate to a simulation or training rather than a dispensing of the material. Thus, instead of dispensing the actual material into the flow channel, knowledge of how this may be performed is stored in the storing means.

Preferably, the flow channel is the same as or of the same type, preferably with the same shape and dimensions, as that into which the material is to be dispensed in order for the flow to be similar or comparable.

Actually, the device may be that of the second embodiment but without the container and with the storing means.

The storing means may be any type of storing means. Also, additional information may be stored therein, such as a total quantity of material to be released, such as a dose. The present aspect is especially relevant in the situation where the rate of release depends on the flow. This may be the situation where the material is provided in a non-pressurized container in which at least a part of the gas flow passes to entrain the material in the gas flow and thus transport the material out of the container. Clearly then, the size of the flow will affect the rate with which the material is entrained and removed from the container.

In some situations, the rate of release also depends on other factors, such as the amount of material still present in the container.

Clearly, also the size/weight/area of the powder may be taken into account when determining the rate of release.

The rate of release may be determined empirically by simply providing a number of the containers and releasing the material sequentially while providing different flows while determining the rate of release, such as over time. Numerical methods may comprise assuming that all material in the container is entrained in the gas in the container. Then, the rate of release depends on the amount of gas entering/exiting the container per unit time. In addition, the concentration may also be taken into account, as it will decrease as material is removed from the container.

The percentage of dispensed material passing the bend is, as described above, depending on e.g. the gas flow. This percentage may be determined for each material or each form it has, as the percentage may depend on also the weight/size/shape/area of the particles. Thus, for a particular material with a particular form (e.g. powder with a particular size/weight distribution), tests may be made for different flow rates of how much material negotiates the bend and how much impacts on the flow channel walls and is adhered thereto. This information may then be stored in the storing means.

Naturally, the calculating means may perform this calculation multiple times, as the flow may vary, and so may the rate of release and thus the percentage. Thus, the calculation may be split up into multiple calculations performed over time, such as periodically, each assuming that the flow, rate of release and percentage is constant for a period of time. This facilitates the calculations.

Naturally, when the flow is determined more or less constantly and the rate of release and percentage are known for many flow values, the calculation may be performed in other manners, but this the skilled person knows.

A final aspect, not forming part of the invention, relates to a computer readable medium having thereon computer executable instructions that, if executed on a computing device, causes the device to:
- receive information relating to a flow of a gas through the flow channel,
- access storing means holding information relating to:
   - a percentage of the gas flow which travels through a container holding the material,
   - a rate of releasing of the material as a function of the gas flow in the channel and
   - a percentage of dispensed material passing the bend as a function of the gas flow in the channel and
- calculate an amount of the material passing the bend on the basis of the actual gas flow in the flow channel, the rate of dispensing at that flow and the percentage at that flow.

Naturally, this aspect may be combined with the other aspects. The computer may simulate or operate both the above inhaler and trainer.

Thus, this may be a storage, such as a Hard drive, a CD, ROM, PROM, EPROM, EEPROM, RAM, flash drive, a tape or any other type of storage holding a program causing the computer to receive information from the flow sensor and access the information of the storing means. Clearly, the storing means may be incorporated in the computer, the calculating means or may be remote therefrom.

The computer will now be able to calculate the amount of the material negotiating the bend from the flow values and the information of the storing means.

### BRIEF DESCRIPTION OF THE DRAWINGS

Embodiments of the invention will now be further described with reference to the drawings, in which:
Fig. 1 illustrates an example of a material, such as a drug, being transported through a bent flow channel, such as the throat of a person,
Fig. 2 illustrates examples of (inhalation) flow rates over time and further a preferred flow interval,
Fig.3 illustrates an embodiment of a delivery device as depicted in Fig. 1, and
Fig. 4 illustrates powder emission at different flow velocities.

### DETAILED DESCRIPTION OF THE DRAWINGS

In general the present invention relates to a method for determining the amount of a powder-shaped material, such as a drug, inhaled by a person and intended to negotiate the bend in the throat of the person.

The invention thus may be used for determining the amount of drug reaching the lungs so as to adapt the dose of the dispenser/container, or it may be used for training the person to optimize the inhalation to ensure that as much as possible of the drug reaches the lungs.

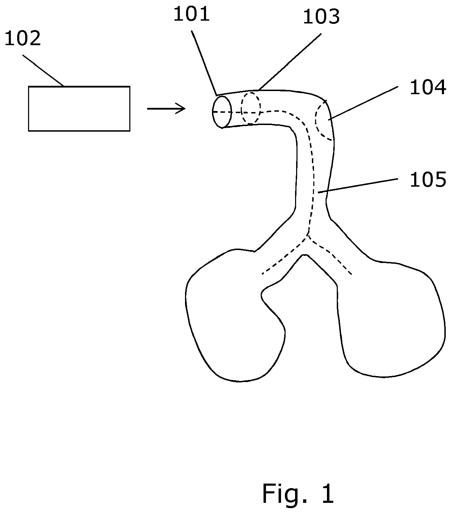

Fig. 1 illustrates a patient 101 and a delivery device 102 configured to deliver a drug to the lungs of the patient 101 via inhalation.

The device 102 is for inhalation, so that the person 101 engages the inhaler with her/his mouth and inhales through the device 102. The inhaled gas/air enters the mouth and, via the bent throat, the lungs.

It is known that if the inhalation flow is too low (Vlow), a too large portion of the drug entrained in the air will impact the surfaces 103 of the mouth and thus not reach the throat or the lungs. If, on the other hand, the inhalation flow is too strong (Vhigh), the drug may not be re-directed in the throat but will impact on the back side 104 of the throat and again not reach the lungs. Thus, a preferred inhalation flow interval exists (Vopt) at which the drug is bent in the throat and remains entrained in the air flow until it reaches the lungs.

Clearly, the preferred inhalation flow interval may depend on the dimensions of the powder as well as the shape and dimensions of the flow channel, including the throat. Thus, for different drugs and/or persons (such as gender and age), different flow intervals may be determined. Often, this correlation is used for identifying a suitable drug and dispenser type for the individual person.

Yet another factor, however, is that the quantity of drug is usually limited. Thus, an unsuitable flow will cause drug to be lost. This cannot be re-gained by prolonging the inhalation at the desired flow.

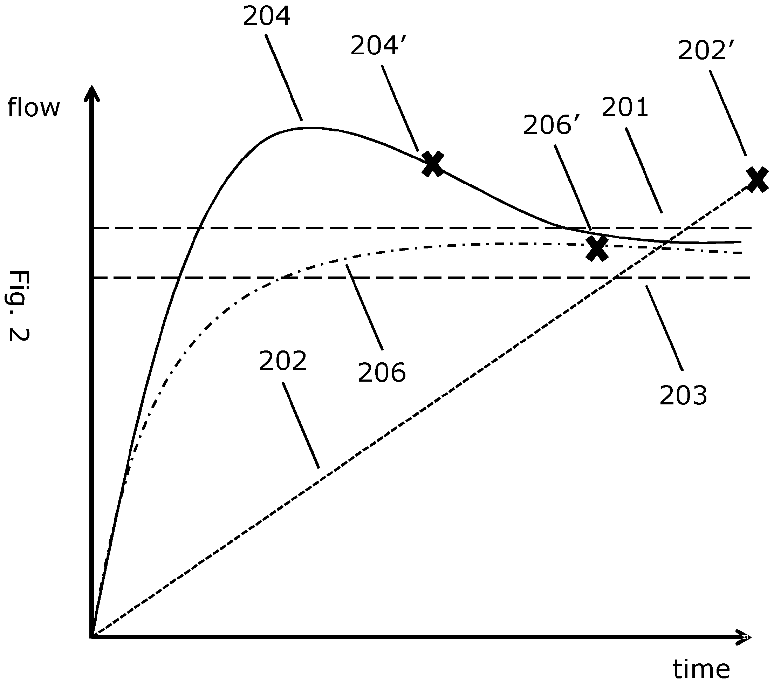

Fig. 2 illustrates three examples of inhalation as a function of time. As previously addressed, an optimal flow rate could ensure a maximum amount of the drug reaching the lungs.

In this example, a preferred flow interval is illustrated by upper and lower boundaries, 201, 203, respectively. In this example, the flow may be the flow velocity of the gas and thus the drug.

A first inhalation 202 is illustrated where the flow increases linearly. The fat X 202' illustrates the point in time where all of the drug has been entrained in the air during the inhalation.

Clearly, most of the time of the inhalation 202 takes place at a too low flow. Thus, a lot of the drug is lost in the mouth of the person. Only for a short period of time is the flow within the interval so that the drug inhaled at this flow actually reaches the lungs.

The second inhalation 204, on the other hand, starts out rather forcefully, so that the flow swiftly increases to become too high. Actually, before the flow is reduced to be within the desired interval, the drug dose is depleted (the fat X 204'), so that only very little drug actually reaches the lungs, even if the flow hereafter stays within the interval for a prolonged period of time.

The third inhalation 206 is more suitable, as the inhalation rather quickly rises to a flow within the interval but stays within the interval until depletion 206'. Clearly, some drug is lost until the flow has increased sufficiently, but the overall amount of drug reaching the lungs of the person in inhalation 206 is much higher than in the other inhalations.

Determination of the amount of drug reaching the lungs thus should take into account the flow over time. Also, the rate of release of the drug, i.e. how much drug is actually entrained in the air, is relevant, as this describes how much drug is presently transported at that flow - and thus how much drug has a too low, too high or suitable velocity on its way to or through the bend.

Clearly, if not all drug is entrained in the air at the same time, this may be taken into account by the rate of release.

Thus, the calculation may be performed in individual steps each based on actual values of the flow and the rate of release. Then, variations in the flow may be taken into account in the determination of the actual amount of drug reaching the lungs. Then, the total amount of drug reaching the lungs (and/or the total amount of drug lost on the way) may be determined by summing these values.

Yet another factor to take into account is the rate of release, which will depend on the type of drug dispensing used.

For powder dispensers, the powder may be provided in single dose blisters or the like which are not under pressure and from which the drug is dispensed by allowing the air to flow through the container and thus mix the air and the drug and in that manner transport the drug out of the container.

Clearly, the rate of release depends on the flow of the gas.

Allowing the gas to flow through a powder capsule will cause the powder to be entrained in the gas and thus be transported out of the capsule. However, if the gas flow is very low, no or only a little powder may be entrained in the gas, whereas for high gas flows, much more powder will be entrained. Thus, the rate of release (the amount of drug per unit time exiting the capsule) will increase with the flow rate - not only due to more gas flowing out of the capsule per unit time but also due to the concentration in the capsule of entrained drug is higher.

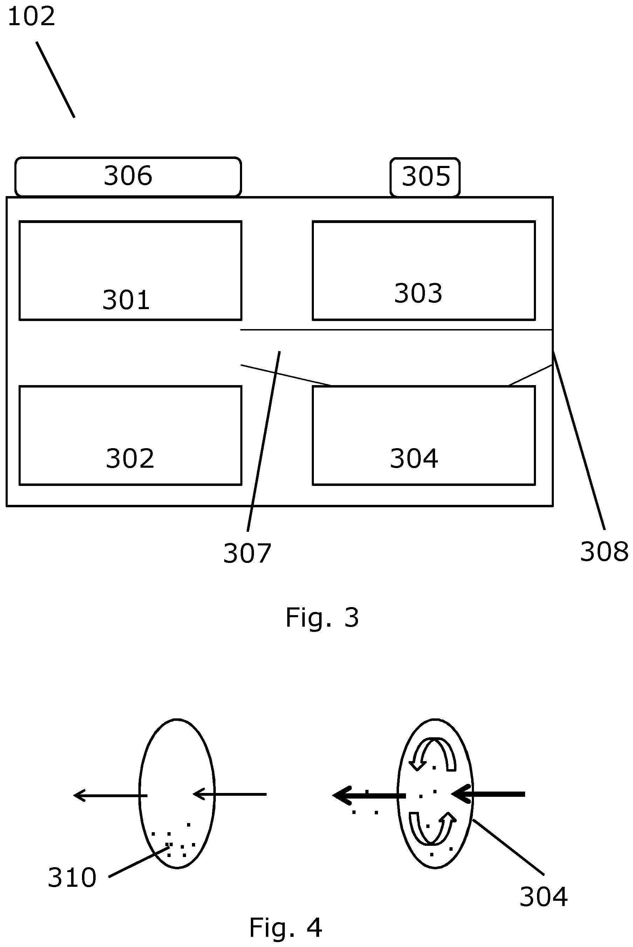

This is illustrated in figure 4 illustrating powder 30 in a drug container 304 where the flow through the container is illustrated by arrows. In the left illustration, the flow through the container is very low, so that the flow in the container is so low that only little powder is entrained in the air. Thus, very little powder, if any, is ejected from the container. In the right illustration, the flow is higher, so that the powder in the container is well entrained in the air and thus ejected from the container with the air.

Thus, the rate of release may also be determined in the calculation.

Clearly, the desired flow interval as well as the rate of release may depend on both parameters of the drug container or delivery system/mechanism as well as of the drug itself (powder size/size distribution/surface/weight and the like) as well as of the flow. These parameters may be determined empirically or numerically.

Fig.3 illustrates the internal components of the delivery device 102 indicated on Fig. 1. The delivery device may comprise a plurality of internal components such as one or more sensors including a flow sensor 301, a data processor 302, a drug dispenser 303, a drug container 304, an actuator 305 and a display 306. In addition, a flow channel 307 is illustrated as well as a mouth piece 308 for the operator to engage during inhalation through the flow channel 307. The flow sensor is positioned so as to determine the flow in the flow channel 307.

The flow sensor 301 may be embodied in a number of manners, such as a rotating element or a pressure sensor may be used to measure the flow during the inhalation time. A variety of additional sensors may be employed such as a temperature sensor, humidity sensor and a strain gauge. A temperature sensor and a humidity sensor may be used to take the environmental factors into account, e.g. calculating the percentage of drug that is lost to the environment or remains in the delivery device 102. A strain gauge may be used to measure the available drug in the drug container 304, especially if this is not a single-use container.

The data processor 302 may be configured to compute the flow rate at a given time instant based on signals provided by the sensor 301. The data processor may also compute a drug dose delivered to the lungs of the patient 101.

All air flow in the flow channel 307 may flow through the container 304, or only a part thereof, where other portions of the flow may flow around the container 304.

The processor 302 may receive information relating to the predetermined quantity of the actual drug as well as the desired flow interval and potentially also the rate of release, such as as a function of time and/or flow rate.

Then, the processor 302 may determine, during or after an inhalation, the total amount of drug reaching the person's lungs from the detected flow and the knowledge relating to the desired flow interval as well as the delivery rate, optionally determined on the basis of the flow and/or time.

This total amount may be output on the display 306. Alternatively, other information may be output such as the flow over time, information of whether the actual or present flow is sufficient, too low or too high, information relating to whether the dose is depleted or how much dose is still available, or the like. Naturally, this information may also be stored in the dispenser and fed to a database for a professional or advisor to review and give advice to the operator.

An actuator 305 may be included. This actuator may be used for operating the dispenser to dispense the dose of the drug, such as rupturing a powder blister. The actuator may additionally or optionally be used for awakening the processor so that the calculation can start. The actuator may be operable by the user by e.g. depressing a button or the like or by the air flow in the channel. The flow sensor may be constantly active and may act to activate the processor and/or dispenser, when the flow reaches a desired level.

Naturally, the device of figure 3 may be altered from being a dispenser to a training device. Thus, the container and the dispenser may be dispensed with. Then, the output of the flow sensor is fed to the processor which then from a storage, which may be provided in the processor, derives information relating to the desired flow interval as well as the rate of delivery, especially if this also depends on the flow rate.

On the basis of this, the processor may again determine how much drug, if it had been dispensed as assumed into the flow channel, would actually reach the uses lungs. Thus, the device is a trainer which may be set to a particular drug, dispenser type or the like (which defines the flow interval and rate of release) so that a user may learn how to inhale in order to, when inhaling through the real inhaler, have as much drug as possible reach the lungs.

## Claims

1. A method for estimating an amount of a powder shaped material passing a bend in a throat of a person, the method comprising the steps of:
- providing a closed, container comprising a predetermined quantity of the powder-shaped material,
- opening the container,
- sensing a flow of a gas, caused by an inhalation of the person, through a flow channel comprising the throat,
- receiving, during a first time interval, information relating to the gas flow, and
- calculating, for a number of points of time during the first time interval:
- a rate of release of the material into the flow channel and
- an amount of the released material having passed the bend,
**characterized in that**:
at least part of the gas flow being provided through the opened container and that
the at least part of the gas flow through the container releases, during the first time interval, the predetermined quantity of the material into the gas flow in the flow channel.

2. The method of claim 1, wherein the step of determining the rate of release of the material into the flow channel comprises basing the determination of the rate of release on the actual flow of the gas in the flow channel.

3. The method of any of the preceding claims, wherein the calculation of the amount of the released material having passed the bend is based on a comparison of the actual flow in the channel to a first flow interval.

4. The method of any of the preceding claims, wherein the calculated amount of material having passed the bend is determined on the basis of the determined rate of release.

5. The method of any of the preceding claims, wherein the calculation is terminated when a predetermined amount of the material has been released into the flow channel.

6. The method of any of the preceding claims, wherein the closed container is non-pressurized.

7. The method of any of the preceding claims, wherein the container is a blister pack.

8. A device (102) for estimating an amount of a powder shaped material passing a bend in a throat of a person (101), the device comprising:
- a closed, container (304) comprising a predetermined quantity of the material, the container being capable of being opened,
- a sensor (301) for determining a flow of a gas through a flow channel (307) comprising the throat of the person,
- opening means (305) capable of opening the container, and
- calculating means (302) for calculating, for a number of points of time during the first time interval:
∘ a rate of release of the material into the flow channel and
∘ an amount of the released material having passed the bend
**characterized in that** the opening means are capable of opening the container so that an inner space of the container comes into fluid connection with the flow channel and forms part of the flow channel to enable releasing, during a first time interval, the predetermined quantity of the material into the gas flow in the flow channel.

9. the device of claim 8, wherein the closed container is non-pressurized.

10. the device of claim 8, wherein the container is a blister pack.

## Patentansprüche

1. Verfahren zum Schätzen einer Menge eines pulverförmigen Materials, das eine Biegung in einem Hals einer Person passiert, wobei das Verfahren die Schritte umfasst:
- Bereitstellen eines geschlossenen Behältnisses, das eine vordefinierte Menge des pulverförmigen Materials umfasst,
- Öffnen des Behälters,
- Erfassen eines durch eine Einatmung der Person verursachten Stroms eines Gases durch einen Strömungskanal, der den Hals umfasst,
- Empfangen von Informationen zu dem Gasstrom während eines ersten Zeitintervalls, und
- Berechnen während des ersten Zeitintervalls für eine Anzahl von Zeitpunkten:
- einer Rate der Freisetzung des Materials in den Strömungskanal, und
- einer Menge des freigesetzten Materials, das die Biegung passiert hat,
**dadurch gekennzeichnet, dass**:
zumindest ein Teil des Gasstroms durch das geöffnete Behältnis bereitgestellt wird und dass
der zumindest eine Teil des Gasstroms durch das Behältnis während des ersten Zeitintervalls die vorbestimmte Menge des Materials in den Gasstrom in dem Strömungskanal freisetzt.

2. Verfahren nach Anspruch 1, wobei der Schritt des Bestimmens der Rate der Freisetzung des Materials in den Strömungskanal das Basieren der Bestimmung der Freisetzungsrate auf den tatsächlichen Strom des Gases in dem Strömungskanal umfasst.

3. Verfahren nach einem der vorstehenden Ansprüche, wobei das Berechnen der Menge des freigesetzten Materials, das die Biegung passiert hat, auf einem Vergleich des tatsächlichen Stroms in dem Kanal zu einem ersten Stromintervall basiert.

4. Verfahren nach einem der vorstehenden Ansprüche, wobei die berechnete Menge an Material, das die Biegung passiert hat, auf Basis der bestimmten Freisetzungsrate bestimmt wird.

5. Verfahren nach einem der vorstehenden Ansprüche, wobei das Berechnen beendet wird, wenn eine vorbestimmte Menge des Materials in den Strömungskanal freigesetzt wurde.

6. Verfahren nach einem der vorstehenden Ansprüche, wobei das geschlossene Behältnis nicht druckbeaufschlagt ist.

7. Verfahren nach einem der vorstehenden Ansprüche, wobei das Behältnis eine Blister-Packung ist.

8. Vorrichtung (102) zum Schätzen einer Menge eines pulverförmigen Materials, das eine Biegung in einem Hals einer Person (101) passiert, wobei die Vorrichtung umfasst:
- ein geschlossenes Behältnis (304), das eine vordefinierte Menge des Materials umfasst, wobei das Behältnis geöffnet werden kann,
- einen Sensor (301) zum Bestimmen eines Stroms eines Gases durch einen Strömungskanal (307), der den Hals der Person umfasst,
- ein Öffnungsmittel (305), das das Behältnis öffnen kann, und
- ein Berechnungsmittel (302) zum Berechnen während des ersten Zeitintervalls für eine Anzahl von Zeitpunkten:
o eine Rate der Freisetzung des Materials in den Strömungskanal, und
o eine Menge des freigesetzten Materials, das die Biegung passiert hat,
**dadurch gekennzeichnet, dass** das Öffnungsmittel das Behältnis öffnen kann, so dass ein Innenraum des Behältnisses mit dem Strömungskanal in Fluidverbindung gelangt und einen Teil des Strömungskanals bildet, um das Freisetzen der vorbestimmten Menge des Materials in den Gasstrom in dem Strömungskanal während eines ersten Zeitintervalls zu ermöglichen.

9. Vorrichtung nach Anspruch 8, wobei das geschlossene Behältnis nicht druckbeaufschlagt ist.

10. Vorrichtung nach Anspruch 8, wobei das Behältnis eine Blister-Packung ist.

## Revendications

1. Procédé d'estimation d'une quantité d'un matériau en forme de poudre passant une courbe dans la gorge d'une personne, le procédé comprenant les étapes :
- de fourniture d'un récipient fermé comprenant une quantité prédéterminée du matériau en forme de poudre,
- d'ouverture du récipient,
- de détection d'un écoulement d'un gaz, provoqué par une inhalation de la personne, à travers un canal d'écoulement comprenant la gorge,
- de réception, pendant un premier intervalle de temps, d'informations relatives à l'écoulement de gaz, et
- de calcul, pour un certain nombre d'instants pendant le premier intervalle de temps :
- d'une vitesse de libération du matériau dans le canal d'écoulement et
- d'une quantité du matériau libéré ayant passé la courbe,
**caractérisé en ce que** :
au moins une partie de l'écoulement de gaz étant fournie par l'intermédiaire du récipient ouvert et **en ce que** l'au moins une partie de l'écoulement de gaz à travers le récipient libère, pendant le premier intervalle de temps, la quantité prédéterminée du matériau dans l'écoulement de gaz dans le canal d'écoulement.

2. Procédé selon la revendication 1, dans lequel l'étape de détermination de la vitesse de libération du matériau dans le canal d'écoulement comprend le fait de baser la détermination de la vitesse de libération sur l'écoulement réel du gaz dans le canal d'écoulement.

3. Procédé selon l'une quelconque des revendications précédentes, dans lequel le calcul de la quantité du matériau libéré ayant passé la courbe est basé sur une comparaison de l'écoulement réel dans le canal à un premier intervalle d'écoulement.

4. Procédé selon l'une quelconque des revendications précédentes, dans lequel la quantité de matériau calculée ayant passé la courbe est déterminée sur la base de la vitesse de libération déterminée.

5. Procédé selon l'une quelconque des revendications précédentes, dans lequel le calcul est terminé lorsqu'une quantité prédéterminée du matériau a été libérée dans le canal d'écoulement.

6. Procédé selon l'une quelconque des revendications précédentes, dans lequel le récipient fermé n'est pas pressurisé.

7. Procédé selon l'une quelconque des revendications précédentes, dans lequel le récipient est un emballage-coque.

8. Dispositif (102) d'estimation d'une quantité d'un matériau en forme de poudre passant une courbe dans la gorge d'une personne (101), le dispositif comprenant :
- un récipient fermé (304) comprenant une quantité prédéterminée du matériau, le récipient pouvant être ouvert,
- un capteur (301) pour déterminer un écoulement d'un gaz à travers un canal d'écoulement (307) comprenant la gorge de la personne,
- des moyens d'ouverture (305) pouvant ouvrir le récipient, et
- des moyens de calcul (302) pour calculer, pour un certain nombre d'instants pendant le premier intervalle de temps :
o une vitesse de libération du matériau dans le canal d'écoulement et
o une quantité du matériau libéré ayant passé la courbe **caractérisé en ce que** les moyens d'ouverture peuvent ouvrir le récipient de sorte qu'un espace interne du récipient entre en communication fluidique avec le canal d'écoulement et fait partie du canal d'écoulement pour permettre une libération, pendant un premier intervalle de temps, de la quantité prédéterminée du matériau dans l'écoulement de gaz dans le canal d'écoulement.

9. Dispositif selon la revendication 8, dans lequel le récipient fermé n'est pas pressurisé.

10. Dispositif selon la revendication 8, dans lequel le récipient est un emballage-coque.
